# EUROPEAN PATENT APPLICATION

(11) **EP 4 295 828 A1**
(43) Date of publication of application: **27.12.2023**
(21) Application number: 23180431.1
(22) Date of filing: 20.06.2023
(51) Int. Cl.: A61F 13/64, A61F 13/551

(54) **REUSABLE BELT FOR ABSORBENT ARTICLE**

(30) Priority: 20.06.2022 IT 202200013015; 18.10.2022 IT 202200021465
(71) Applicant: Fippi SpA, 20123 Milano (MI) (IT)
(72) Inventor: GUARNERIO, Lorenzo, 20123 Milano (MI) (IT); GUARNERIO, Claudio, 20123 Milano (MI) (IT); TANASE, Elena, 20123 Milano (MI) (IT); MERONI, Massimiliano, 20123 Milano (MI) (IT); BAX, Serge, 20123 Milano (MI) (IT)
(74) Representative: Ercolani, Simone Pietro

(57) **Abstract**

Reusable belt (1) for absorbent article (2) comprising a central portion (10) which can be reversibly combined with said absorbent article, a first side portion (11) jutting from a first edge (10) of said central portion (10) and a second side portion (12) jutting from a second edge (10b) of said central portion (10), which is opposite said first edge (10a), said belt (1) further comprising means (13) for reversibly constraining said first side portion (11) to said second side portion (12), at least when said first side portion (11) and said second side portion (12) encircle the user's waist, characterised in that said first side portion (11) and/or said second side portion (12) are made of a material distinct from that of which said central portion (10) is made, and in that said first side portion (11) and/or said second side portion (12) is/are integrally constrained to said first edge (10a) and/or said second edge (10b) of said central portion (10), along the respective side connecting edge (11a,12a).

## Description

### FIELD OF THE INVENTION

The present invention concerns a belt for absorbent article.

### KNOWN PRIOR ART

Reusable belts are known to hold absorbent articles in position when in use.

For example, the patent EP0409307A2 in the name of Procter & Gamble Company describes a garment for collecting body dejections consisting of a belt and a disposable assembly that is removably attached to the belt. The belt can be attached and detached from this disposable assembly by means of a removable attachment of the hook and loop type (i.e., by means of a Velcro^{®} system). The belt is shaped so that it can be reused.

EP 0605014 A1 in the name of KIMBERLY-CLARK describes a disposable absorbent garment that provides for pleated straps to enable the garment to be worn. The straps can be removably constrained at the ends of the absorbent garment by means of buttons or Velcro^{®}-type material.

The pleating of the straps, which can be adjusted, has the purpose of fitting the straps to the user of the absorbent garment.

GB 2242612 A in the name of KIMBERLY CLARK CO describes a disposable diaper equipped with a stretch belt that can be removably attached to the diaper.

In particular, the belt may be inserted into slots arranged at an end of the diaper and may be equipped with a Velcro^{®}-type material at the ends of the belt to allow the closure of the same belt at the waist of the diaper user.

JP2011172793A in the name of LIVEDO CORPORATION describes a belt element for a diaper that is washable and reusable. This belt element can be removably attached to a disposable diaper through a Velcro^{®}-type material.

US 4,964,860 in the name of THE PROCTER & GAMBLE COMPANY describes an absorbent garment that consists of an absorbent portion, of the disposable type, and a reusable belt, which can be constrained to each other by means of a hook and loop attachment.

WO 2009/043101 A1 in the name of KUVER DESIGNS PTY LTD relates to a diaper comprising a body portion of the disposable type, and a detachable and reusable belt.

A Velcro^{®}-type material is used to couple the belt to the body portion.

WO 2013/012374 A1 in the name of SCA HYGIENE PROD AB relates to a package comprising a certain number of diapers and a reusable belt that is removably attached to each diaper by means of Velcro^{®}. The belt can act as a gripping device for at least one diaper arranged within the aforesaid package.

JP2011167352A in the name of DAIEI KK refers to a belt for fastening a pad and sanitary products that fit patients with different physical dimensions and that are used for treating lochia after childbirth and treating excretion during the hospitalisation and the like.

US2019021915A1 in the name of HOONCOOP et Alii refers in general to diapers and, in particular, to devices to prevent the removal of a diaper by a child wearing the diaper.

While such solutions are useful to allow at least the reuse of part of a diaper (i.e. the reusable belt) and thus to limit the causes of pollution resulting from the use of such products, they do not always prove to be comfortable for the user. The reusable belt often does not specifically fit the shape of the user's pelvis, either by tightening too much on the user's body or by being too wide and thus leaving the absorbent article free to move excessively.

In light of the above, object of the present invention is to make a reusable belt for absorbent article that can easily fit the shape of the user's pelvis.

Additionally, object of the present invention is to make a reusable belt that can also be easily made.

Finally, object of the present invention is to make a diaper that implements the use of such a reusable belt.

### SUMMARY OF THE INVENTION

These and other objects are achieved by means of a reusable belt according to the first independent claim of the present invention.

In particular, the objects are achieved by means of a belt for absorbent article comprising a central portion that can be reversibly combined with said absorbent article, a first side portion jutting from a first edge of said central portion and a second side portion jutting from a second edge of said central portion, opposite said first edge, said belt further comprising means for reversibly constraining said first side portion and said second side portion to said absorbent article, characterised in that said central portion is made of a material having elastic capabilities lower than those of the material of which said first side portion and/or said second side portion is/are made.

This solution allows, therefore, to solve the problems of known art. In fact, the two side portions can extend much more than the central portion, thus allowing the dimensions of the two side portions of the belt to be able to be adjusted at will, as needed, i.e. depending on the dimensions of the user's pelvis.

In particular, said first side portion and/or said second side portion is/are made of a generally elastic material.

In addition, the material of said first side portion and said second side portion comprises a first fabric which comprises fibres of elastic material and fibres of inelastic material, preferably the elastic material fibres comprise elastane, and even more preferably the inelastic material fibres comprise one of polyamide, cotton, polypropylene or polyester, or a combination thereof.

Furthermore, said first side portion and/or said second side portion is/are extendable by a value higher than 5 mm, preferably higher than 15 mm.

Additionally, said central portion, if at all, is made of an inelastic material, i.e. having elastic and, therefore, elongation capabilities, close to zero.

In particular, the material of said central portion comprises a first fabric, which comprises elastic material fibres and inelastic material fibres, preferably the elastic material fibres comprise elastane and, even more preferably, inelastic material fibres comprise one of polyamide, cotton, polypropylene and polyester or a combination thereof, and a second fabric joined overlapping said first fabric at said central portion, which comprises inelastic material fibres. This second fabric preferably comprises fibres of polyamide, cotton, polypropylene, polyester or a combination thereof.

The second fabric can be joined to the first fabric by a high-tech machine sewing operation (e.g. for manufacturing socks) with yarns (cotton, polyamide, Lycra or other material used in these types of production). The second fabric allows to limit, if not zero, the elastic capabilities of the first fabric.

Additionally, the sewing may take place by ultrasound.

Finally, the sewing may take place by a closing system of the Velcro, buttons or glue type.

In addition, the first fabric can have the following composition:
- about 37% elastane fibres, 35% polyamide fibres and 28% cotton fibres for an area weight of about 375 gsm; or
- about 62% cotton fibres, 38% elastane fibres; or
- about 37% elastane fibres, 37% polyamide fibres and 26% polyester fibres for an area weight of about 375 gsm; or
- about 61% polyester fibres and 39% elastane fibres for an area weight of about 350 gsm.

In accordance with a first embodiment of the invention, said first fabric of said first side portion, of said second side portion and said central portion is made of a single piece. In this case, therefore, once this single piece of the first fabric is properly shaped, the second fabric is combined with the first fabric at the central portion in order to make this central portion perfectly inelastic.

In accordance with an alternative embodiment, said first side portion and/or said second side portion is/are integrally constrained to said first edge and/or said second edge of said central portion, along the respective side connecting edge.

In this case, therefore, there are three distinct pieces of first fabric that are each shaped appropriately to make the central portion and the two side portions.

Furthermore, said first side portion and said second side portion are joined to the connecting edges of said central portion by sewing, along the respective side connecting edge.

This sewing operation may take place by means of a tailoring machine.

In another embodiment, this sewing operation may take place by means of a high-tech machine (e.g. for manufacturing socks) with yarns (cotton, polyamide, Lycra or other material used in these types of production).

Additionally, the sewing may take place by ultrasound.

Finally, the sewing may take place by a closing system of the Velcro, buttons or glue type.

According to an alternative embodiment, said means for reversibly constraining said first side portion and said second side portion to said absorbent article comprise at least one first tear-off fastening element, e.g. of the Velcro^{®} type, and a second tear-off fastening element, e.g. of the Velcro^{®} type; said first tear-off fastening element is combined with an end region of said first side portion and said second tear-off fastening element is combined with an end region of said second side portion. The first tear-off fastening element and the second tear-off fastening element are used to close the two side portions on the absorbent article around the user's pelvis. In practice, the first fastening element and the second fastening element are combined with the absorbent article (in particular, its front portion) when the first and second side portions encircle together the user's pelvis.

In accordance with a further embodiment, said means for reversibly constraining said first side portion and said second side portion to said absorbent article comprise at least one lace combined with said first portion at a first end thereof, at least one third tear-off fastening element integrally constrained to a second free end of said at least one lace and at least one fourth tear-off fastening element integrally constrained to said second side portion on the outer side of said second side portion, said third tear-off fastening element being equipped with an active surface positioned on the side of the lace facing the inner side of said first side portion, wherein said fourth tear-off fastening element is equipped with an active surface that can be coupled to the active surface of said third tear-off fastening element.

Furthermore, said lace is made of elastic material.

Additionally, the reusable belt comprises further means for reversibly combining said central portion with said absorbent article, wherein said further means comprise one or more tear-off fastening portions constrained to said central portion.

This one or more tear-off fastening portions are, e.g., of Velcro^{®} type, to combine said absorbent article (in particular, its back portion) with said central portion.

The objects are also achieved by a method for making a reusable belt according to one or more of claims 1 to 10, comprising the step a) of conforming to size said central portion, said first side portion and said second side portion, wherein said central portion is made of a material having elastic capabilities lower than those of the material of which said first side portion and/or said second side portion is/are made.

In particular, in accordance with a first embodiment of the invention, said step a) comprises step a1) of starting from a single piece of a first fabric and step a2) of cutting to size said single piece of first fabric to obtain said central portion, said first side portion and said second side portion, and in that it comprises step b) of integrally combining said second fabric with said first fabric, at said central portion. In practice, therefore, according to this embodiment, a single piece of first fabric is used which, without being further divided, is cut to size to obtain said central portion, said first side portion and said second side portion. The belt, therefore, comprises a single layer of first fabric and the two side portions and the central portion are connected to each other in one piece, by means of this first fabric.

In accordance, on the contrary, with a second embodiment of the invention, said step a) comprises the step a1') of starting from three distinct pieces of a first fabric and the step a2') of cutting to size each piece of said three pieces of said first fabric to obtain said central portion, said first side portion and said second side portion, and in that it comprises step the b) of integrally combining said second fabric with said first fabric, at said central portion, and step d) of integrally constraining said first side portion and/or said second side portion to said first edge and/or to said second edge of said central portion, along the respective side connecting edge. In this embodiment, unlike the one described above, the two side and central portions constitute three distinct and separate elements which are joined together during step d). This step d) is in fact absent in the previous embodiment of the invention described above.

In the two distinct embodiments described above, said step b) of combining said second fabric with said first fabric has the task of reducing the elastic capabilities of the first fabric at the central portion, and thus of making the material, of which the central portion consists, inelastic or with reduced elastic capabilities, overall.

Eventually, said step b) and/or said step d) comprise/comprises the step c) of sewing, respectively, said second fabric to said first fabric and/or said first side portion and/or said second side portion to said central portion.

This sewing operation may take place by means of a high-tech machine (e.g. for manufacturing socks) with yarns (cotton, polyamide, Lycra or other material used in these types of production).

Additionally, the sewing may take place by ultrasound.

Finally, the sewing may take place by a closing system of the Velcro, buttons or glue type.

Additionally, the invention provides a diaper comprising an absorbent article and a reusable belt according to one or more of claims 1 to 10, wherein said reusable belt can be reversibly combined with said absorbent article.

In particular, said absorbent article comprises a permeable upper web, a waterproof lower web and an absorbent core comprising absorbent material arranged between an upper layer and a lower layer. Said absorbent core is arranged between said upper web and said lower web, wherein the absorbent core preferably comprises one or more attaching areas, wherein the upper layer is combined with the lower layer and wherein said one or more attaching areas are devoid of said absorbent material.

Finally, the absorbent article comprises at least one adhesive band arranged on the outer surface of said lower web; said at least one adhesive band is covered by at least one removable protection strip. This solution allows the absorbent article to be able to be closed on itself, so as to contain the dejections therein, before being discarded by the user. In fact, the presence of the belt eliminates the need to provide, on the sides of the absorbent article, at least one of the two pairs of common adhesive tabs adapted to close the absorbent article once it has fulfilled its task. It is known, in fact, that normal diapers have four adhesive tabs arranged two by two on the longitudinal opposite sides of the diaper, at the back and the front, respectively. In the case of the solution described herein, however, the belt avoids the use of the traditional pair of tabs arranged at the back, where the belt is now positioned. This way, in the absence of the aforementioned adhesive band, the absorbent article would be difficult to close once used.

### BRIEF DESCRIPTION OF THE FIGURES

These and other aspects of the present invention will be made clearer by the following detailed description of a preferred embodiment provided herein only by way of nonlimiting example, with reference to the accompanying figures, wherein:
Figure 1 is a plan view of the reusable belt according to a first embodiment of the invention;
Figure 2A is a plan view of the belt in accordance with a second embodiment of the invention;
Figure 2B is a plan view of the belt of figure 2A, in which the two side portions and the central one are still separated;
Figure 3 is a plan view of the reusable belt of figure 1, combined with an absorbent article to form a diaper;
Figure 4 is a cross-sectional view of the absorbent article shown in figure 3, along the adhesive band combined with the outer surface of the same absorbent article;
Figure 5 is a plan view of the reusable belt in accordance with a further embodiment of the invention;
Figure 6 is a plan view of the reusable belt on its inner side, in accordance with a further embodiment of the invention;

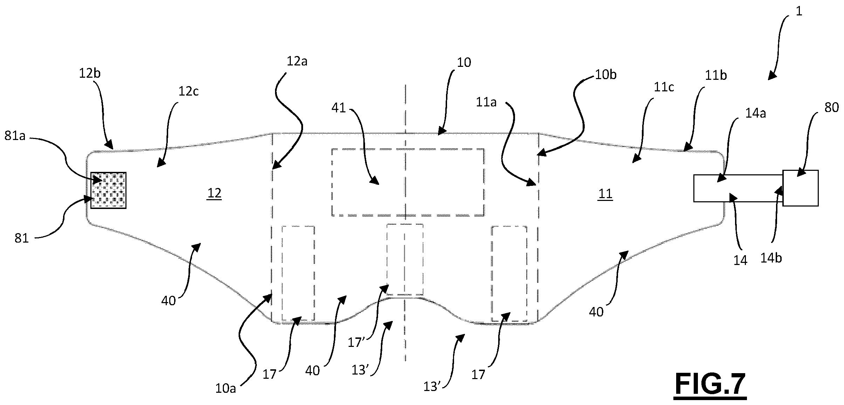

Figure 7 is a plan view of the reusable belt according to figure 6, on its outer side.

### DETAILED DESCRIPTION OF A PREFERRED EMBODIMENT OF THE PRESENT INVENTION

With particular reference to these figures, 1 denotes a reusable belt according to the invention.

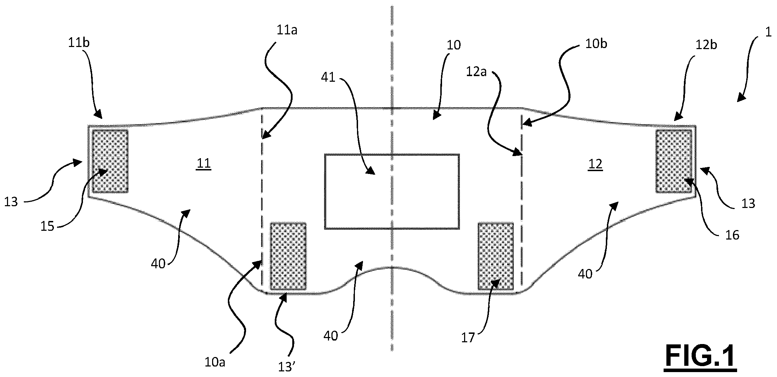

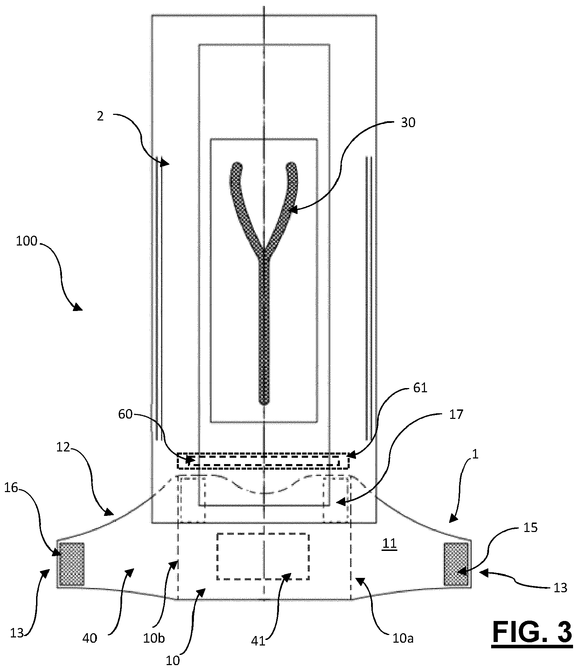

The reusable belt 1 for absorbent article 2 (see figure 1 and figure 3) comprises a central portion 10 which can be reversibly combined with the absorbent article 2, a first side portion 11 jutting from a first edge 10a of the central portion 10 and a second side portion 12 jutting from the second edge 10b of the central portion 10. This second edge 10b is opposite the first edge 10a. The belt 1 further comprises means 13 for reversibly constraining the first side portion 11 and the second side portion 12 to the absorbent article 2, at least when the first side portion 11 and the second side portion 12 encircle the user's waist.

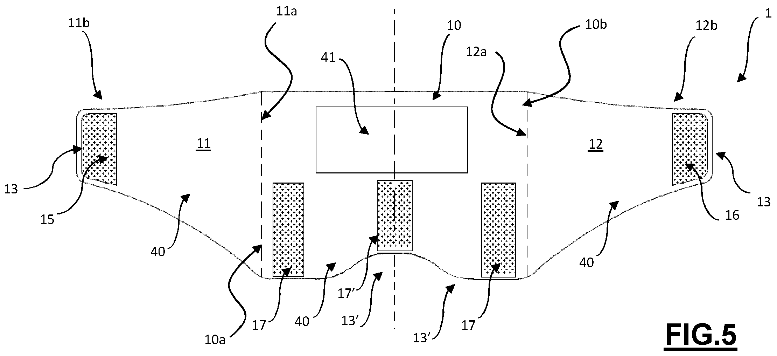

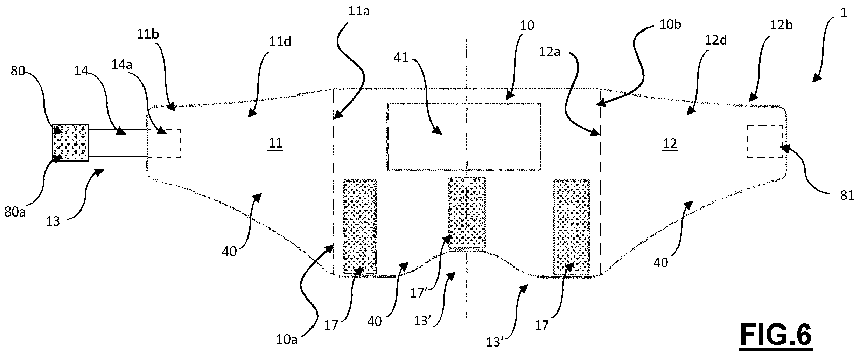

In a particular embodiment shown in figures 5 and 6, these means 13 for reversibly constraining the first side portion 11 and the second side portion 12 to the absorbent article 2 comprise a lace 14 combined, e.g. by sewing, with the first portion 11 at a first end 14a thereof, a third tear-off fastening element 80 integrally constrained to a second free end 14b of the lace 14 and a fourth tear-off fastening element 81 integrally constrained to the second side portion 12. In particular, the fourth tear-off fastening element 81 is positioned on the outer side 12c of the second side portion 12 (opposite the inner side 12d), whereas the active surface 80a of the third tear-off fastening element 80, i.e. the surface that is combined with the corresponding active surface 81a of the fourth tear-off fastening element 81, is positioned on the side of the lace 14 that faces the inner side 11d of the first side portion 11, which is opposite the outer side 11c. In particular, the fourth element 81 is equipped with an active surface 81a that can be coupled to the active surface 80a of the third tear-off fastening element 80.

It should be noted that inner side of the first side portion 11, or of the second side portion 12 or of the central portion 10, means that side of the belt 1 which, when worn by the user, faces the same user.

The lace 14 can be sewn to the first portion 11 at either the inner side 11d or the outer side 11c of that first portion 11. In the embodiment shown herein and represented in figures 5 and 6, this lace 14 is sewn on the outer side 11c of the first portion 11.

Said third tear-off element 80 and said fourth tear-off element 81 are of the Velcro^{®} or, alternatively, button type.

Furthermore, the lace 14 is made of elastic material. In any case, in other embodiments, this lace 14 can be made of a flexible but not extensible material, without thereby departing from the protection scope of the present invention.

According to the embodiment described herein, the first side portion 11 and the second side portion 12 are made of an elastic material, whereas the central portion 10 is made of an inelastic material, i.e. with elastic capabilities close to zero. Also falling within the invention is a solution in which the central portion 10 has a reduced elastic capability compared to that of the two side portions 11 and 12.

This way, the two side portions 11 and 12 are extendable, while the central portion 10 is not. Thanks to this feature, it will be possible to fit the length of the two side portions 11,12 more easily to the dimensions of the user's pelvis, thus identifying the most comfortable size for the user.

It should be noted that absorbent article 2 means a product capable of absorbing the user's dejections.

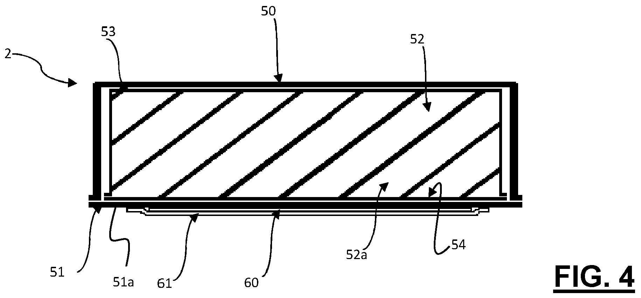

In particular, this absorbent article 2 comprises, in a known manner (see figure 4 in this regard), a permeable upper web 50, a waterproof lower web 51 equipped with an outer surface 51a, an absorbent core 52 comprising absorbent material 52a arranged between an upper layer 53 and a lower layer 54. The absorbent core 52 is arranged between the upper web 50 and the lower web 51.

Preferably, the absorbent core 52 comprises one or more attaching areas 30 (see figure 3 in this regard) in which the upper layer is combined with the lower layer; such one or more attaching areas 30 are free of the absorbent material. In the embodiment shown in figure 3, there is only one Y-shaped attaching area, or channel, 30. The absorbent material 52a is made, in a known way, by using fluff material, typically cellulose fluff pulp. However, in other embodiments, the absorbent core may be substantially flufffree and comprise superabsorbent polymers also known by the acronym SAP. In addition, the absorbent core 52 may comprise a combination of cellulose fluff pulp and superabsorbent polymers (SAP). These superabsorbent polymers refer to any suitable particulate (e.g., in flakes, particulate, granule or powder) or fibrous cross-linked polymer materials that can absorb at least 5 times and preferably at least about 10 times or more its weight of a 0.9% aqueous saline solution as measured by using the Centrifuge Retention Capacity Test (EDANA 441.2-01).

Advantageously, the first side portion 11 and the second side portion 12 are made of a material distinct from the material of which the central portion 10 is made.

In accordance with the embodiment shown in figure 1, the material of the first side portion 11 and the second side portion 12 comprises a first fabric 40 which comprises fibres of elastic material and fibres of inelastic material, preferably the elastic material fibres comprise elastane and, even more preferably, the inelastic material fibres comprise one of polyamide, cotton, polypropylene, polyester or a combination thereof.

In particular, in the specific embodiment described herein, the material of the two side portions 11 and 12 comprises a first fabric 40 which, in turn, comprises about 37% elastane fibres, 35% polyamide fibres and 28% cotton fibres for an area weight of about 375 gsm. However, in another embodiment, the percentages of elastic and inelastic fibres and the type of material used may vary without thereby departing from the protection scope of the present invention.

With this composition, the first side portion 11 and the second side portion 12 are extendable by a value higher than 15 mm.

In any case, an embodiment in which the first fabric 40 of the first side portion 11 and/or the second side portion 12 has a composition different from that mentioned above and, therefore, involves an elongation value higher than 5 mm compared to the starting value, would still fall within the scope of the protection of the present invention.

Another type of first fabric 40 could, however, be used to have an elongation of 15 mm, or another value, compared to the starting measure, without thereby departing from the protection scope of the present invention.

In addition, the central portion 10, as mentioned above, is made of an inelastic material.

In particular, the material of the central portion 10 comprises the same first fabric 40 as described for the two side portions 11 and 12, which comprises fibres of elastic material and fibres of inelastic material, preferably the elastic material fibres comprise elastane and, even more preferably, the inelastic material fibres comprise one of polyamide, cotton, polypropylene and polyester or a combination thereof, and a second fabric 41 joined integrally overlapping the first fabric 40 at the central portion 10, which comprises inelastic material fibres. This second fabric 41 preferably comprises fibres chosen from polyamide, cotton, polypropylene and polyester or a combination thereof.

In the embodiment described herein, this operation of joining the second fabric 41 to the first fabric 40 is carried out by sewing and takes place by tailoring machine.

In another embodiment, this sewing operation may take place by means of a high-tech machine (e.g. for manufacturing socks) with yarns (cotton, polyamide, Lycra or other material used in these types of production). Additionally, the sewing may take place by ultrasound or a closing system of the Velcro, buttons or glue type, without thereby departing from the protection scope of the present invention.

According to the embodiment shown in figure 1, the first fabric 40 of the first side portion 11, the second side portion 12 and the central portion 10 is made of a single piece, therefore, the two side portions 11 and 12 and the central portion 10 are joined without interruptions thanks to the first fabric 40.

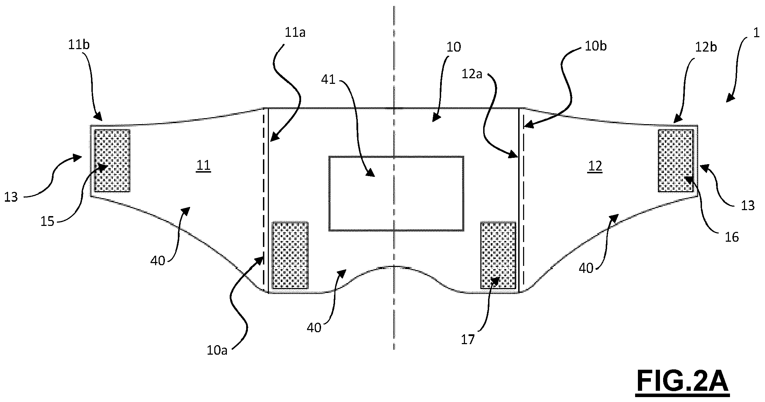

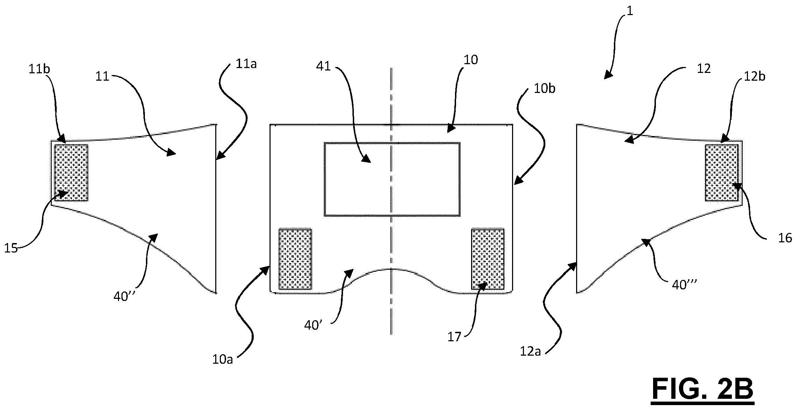

In figures 2A and 2B, however, a further embodiment of the invention is shown, wherein the first side portion 11 and the second side portion 12 are integrally constrained to the first edge 10a and the second edge 10b of the central portion 10, along the respective side connecting edge 1 1a,12a present at the first side portion 11 and the second side portion 12.

According to a particular aspect of the invention, the first side portion 11 and the second side portion 12 are joined to the connecting edges 10a, 10b of the central portion 10 by sewing, along the respective side connecting edge 11a, 12a.

In the embodiment described herein, this sewing operation takes place by tailoring machine.

In another embodiment, this sewing operation may take place by means of a high-tech machine (e.g. for manufacturing socks) with yarns (cotton, polyamide, Lycra or other material used in these types of production). Additionally, the sewing may take place by ultrasound or a closing system of the Velcro, buttons or glue type, without thereby departing from the protection scope of the present invention.

Furthermore, in a further embodiment distinct from that shown in figures 5 and 6, the means 13 for reversibly constraining the first side portion 11 and the second side portion 12 to the absorbent article 2 comprise at least one first Velcro^{®} fastening element 15 and at least one second Velcro^{®} fastening element 16, respectively. This first Velcro^{®} element 15 is combined with an end region 1 1b of the first side portion 11 opposite to the edge 11a and the second Velcro^{®} element 16 is combined with an end region 12b of the second side portion 12, which is opposite to the edge 12a.

In practice, the first fastening element 15 and the second fastening element 16 are combined with the absorbent article, in particular with its front portion, when the first and second side portions 11 and 12 encircle together the user's pelvis. The two fastening portions 15 and 16 are combined with the outer surface 51a of the lower web 51 of the absorbent article 2. In particular, they are combined with the front portion of the absorbent article 2, which portion is intended to cover the front region of the user.

The belt 1 further comprises additional means 13' for reversibly combining the central portion 10 with the absorbent article 2. These additional means 13' comprise two Velcro^{®} tear-off fastening portions 17 constrained to the central portion 10 for combining the absorbent article 2 with the central portion 10 of the belt 1.

This one or more tear-off fastening portions are of the, e.g., Velcro^{®} type for combining the absorbent article 2 with the central portion 10.

These two Velcro fastening portions 17 are arranged on the two sides (thus they are side fastening portions) of the central portion 10 in the proximity of the first edge 10a of the central portion 10 and the second edge 10b of the central portion 10, respectively.

In practice, these further removable fastening means 13', constrained to the central portion 10, are combined with the absorbent article 2, in particular with its back portion, when the absorbent article is arranged on the belt 1. The two fastening portions 17 are combined with the outer surface 51a of the lower web 51 of the absorbent article 2. In particular, they are combined with the back portion of the absorbent article 2, which is intended to cover the back region of the user.

Figure 5 shows a further embodiment of the belt 1 which can be made in any one of the embodiments described above and which, however, it is distinguished from these in that the further reversible fastening means 13' comprise a further fastening portion 17' arranged constrained at the centre of the central portion 10 symmetrically to the two side fastening portions 17 described above.

The waterproof outer surface 51a of the upper web 51 is made of a material such that it is possible to fasten the absorbent article 2 to those removable fastening means 13 and/or to those further removable fastening means 13'.

Figure 3 shows a diaper 100 according to the invention.

This diaper 100 comprises an absorbent article 2 and the reusable belt 1 as described above, in particular as shown in figure 1 or, in any case, according to one or more of claims 1 to 10. The reusable belt 1 can be reversibly combined with the absorbent article 2 by means of two Velcro^{®} fastening portions 17 for combining the absorbent article 2 with the central portion 10. The number of these fastening portions 17 may vary without thereby departing from the protection scope of the present invention. For example, figure 3 shows a belt 1 comprising three fastening portions 17 and 17' which, compared to the embodiment in figure 1 or 3, has more retention capability than the absorbent article 2.

In particular, as already mentioned above, the absorbent article 2 comprises a permeable upper web 50, a waterproof lower web 51, an absorbent core 52 comprising absorbent material 52a arranged between the upper layer 53 and the lower layer 54.

The absorbent core 52 is arranged between the upper web 50 and the lower web 51, wherein the absorbent core 52 preferably comprises a "Y"-shaped attaching area 30 in which the upper layer 50 is directly combined with the lower layer 51. In practice, the "Y"-attaching area 30 is devoid of the absorbent material.

Finally, the absorbent article 2 comprises an adhesive band 60 arranged on the outer surface 51a of the lower web 51. This adhesive band 61 is completely covered by a removable protection strip 61. Once the diaper 1 has been used, the belt 2 has been removed from the absorbent article 2 and the protective strip 61 has been taken off, it will then be possible to roll up the absorbent article 2 and keep it closed on itself before finally disposing of it. This solution therefore allows, once the diaper 100 has been used and the belt 1 has been removed, the absorbent article 2 to be able to be closed with the dejections contained therein, thus preventing it from being subsequently reopened. In fact, the presence of the belt 1 eliminates the need to provide, on the sides of the absorbent article 2, the two pairs of common adhesive tabs adapted to close the absorbent article once it has been used. It is known, in fact, that normal diapers have four adhesive tabs arranged two by two on the longitudinal opposite sides of the diaper, at the back and the front, respectively. However, in the case of the solution described herein, the belt 1 would prevent the use of the aforementioned traditional pairs of tabs arranged at the back. In place of these, however, the adhesive band 60 covered by the protection strip 61 is provided, which is removed when necessary, i.e. when the absorbent article 2 should be rolled up on itself at the end of its use.

It should be noted that the outer surface 5 1a of the lower web 51 refers to the surface which is opposite the surface in contact with the absorbent core 52 and which is, therefore, in contact with the external environment.

Below, the method for making the reusable belt 1 described above is also described in accordance with the two embodiments described above.

This making method comprises the step a) of conforming to size the central portion 10, the first side portion 11 and the second side portion 12, wherein the central portion 10 is made of a material having elastic capabilities lower than those of the material of which the first side portion 11 and the second side portion 12 are made. In particular, the material of the two side portions 11 and 12 is elastic, whereas the material of the central portion 10 is inelastic.

In accordance with figure 1, step a) comprises step a1) of starting from a single piece of a first fabric 40 and step a2) of cutting this single piece of the first fabric 40 to size to obtain the central portion 10, the first side portion 11 and the second side portion 12. This way, the central portion 10, the first side portion 11 and the second side portion 12 are joined in one piece without interruptions. In addition, the method comprises the step b) of integrally combining the second fabric 41 with the first fabric 40, at the central portion 10. This allows to make the central portion 10 inelastic or with little elastic capabilities compared to the two side portions 11 and 12.

Alternatively, as shown in figure 2, the step a) comprises the step a1') of starting from three distinct pieces 40',40",40" of a first fabric 40 and the step a2') of cutting to size each piece 40',40",40" of the three pieces of first fabric 40 to obtain the central portion 10, the first side portion 11 and the second side portion 12. According to this embodiment, therefore, the central portion 10, the first side portion 11 and the second side portion 12 are physically separated from each other.

In addition, the method also comprises the step b) of integrally combining the second fabric 41 with the first fabric 40 at the central portion 10, and the step d) of integrally constraining the first side portion 11 and the second side portion 12 to the first edge 10a and the second edge 10b of the central portion 10, along the respective side connecting edge 11a, 12a. Thanks to step b), the central portion 10 is made inelastic or, in any case, with little elastic capabilities compared to those of the two side portions 11 and 12.

Step b) may also take place after step d), without thereby departing from the protection scope of the present invention.

Finally, step b) and step d) comprise the step c) of sewing the second fabric 41 to the first fabric 40 and/or the first side portion 11 and/or the second side portion 12 to the central portion 10.

This sewing step c) may take place by means of tailoring machine.

In another embodiment, this sewing step c) may take place by means of a high-tech machine (e.g. for manufacturing socks) with yarns (cotton, polyamide, Lycra or other material used in these types of production). Additionally, this sewing step c) may take place by ultrasound or by means of a closing system of the Velcro, buttons or glue type.

## Claims

1. Reusable belt (1) for absorbent article (2), comprising a central portion (10) that can be reversibly combined with said absorbent article, a first side portion (11) jutting from a first edge (10a) of said central portion (10) and a second side portion (12) jutting from a second edge (10b) of said central portion (10), opposite said first edge (10a), said belt (1) further comprising means (13) for reversibly constraining said first side portion (11) and said second side portion (12) to said absorbent article (2), at least when said first side portion (11) and said second side portion (12) encircle the user's waist, **characterised in that** said central portion (10) is made of a material having elastic capabilities lower than those of the material of which said first side portion (11) and/or said second side portion (12) is/are made.

2. Belt (1) according to claim 1, **characterised in that** the material of said first side portion (11) and said second side portion (12) comprises a first fabric (40) which comprises fibres of elastic material and fibres of inelastic material, preferably the elastic material fibres comprise elastane, and even more preferably the inelastic material fibres comprise polyamide, cotton, polypropylene and/or polyester.

3. Belt (1) according to claim 1 or 2, **characterised in that** said first side portion (11) and/or said second side portion (12) is/are extendable by a value higher than 5 mm, preferably higher than 15 mm.

4. Belt (1) according to one or more of claims 1 to 3, **characterised in that** said central portion (10) is made of an inelastic material.

5. Belt (1) according to claim 4, **characterised in that** the material of said central portion (10) comprises a first fabric (40) which comprises fibres of elastic material and fibres of inelastic material, preferably the elastic material fibres comprise elastane and, even more preferably, the inelastic material fibres comprise polyamide, cotton, polypropylene and/or polyester, and a second fabric (41), joined overlapping said first fabric (40) at said central portion (10), which comprises inelastic material fibres, this second fabric preferably comprising fibres of polyamide, cotton, polypropylene and/or polyester.

6. Belt according to one or more of claims 2 to 5, **characterised in that** said first fabric (40) of said first side portion (11), of said second side portion (12) and of said central portion (10) is made in one piece.

7. Belt (1) according to one or more of claims 1 to 5, **characterised in that** said first side portion (11) and/or said second side portion (12) is/are integrally constrained to said first edge (10a) and/or to said second edge (10b) of said central portion (10), along the respective side connecting edge (11a, 12a).

8. Belt according to claim 7, **characterised in that** said first side portion (11) and said second side portion (12) are joined to the connecting edges (10a, 10b) of said central portion (10) by sewing, along the respective side connecting edge (11a, 12a).

9. Belt according to one or more of claims 1 to 8, **characterised in that** said means (13) for reversibly constraining said first side portion (11) and said second side portion (12) to said absorbent article (2) comprise at least one lace (14) combined with said first portion (11) at a first end (14a) thereof, at least one third tear-off fastening element (80) integrally constrained to a second free end (14b) of said at least one lace (80) and at least one fourth tear-off fastening element (81) integrally constrained to said second side portion (12) on the outer side (12c) of said second side portion (12), said third tear-off fastening element (80) being equipped with an active surface (80a) positioned on the side of the lace (14) which faces the inner side (11d) of said first side portion (11), said fourth tear-off fastening element (81) being equipped with an active surface (81a) that can be coupled to the active surface (80a) of said third tear-off fastening element (80).

10. Belt according to claim 9, **characterised in that** said lace is made of elastic material.

11. Belt according to one or more of claims 1 to 10, **characterised by** comprising further means (13') for reversibly combining said central portion (10) with said absorbent article (2), said further fastening means (13') comprising one or more tear-off fastening portions (17,17') constrained to said central portion (10).

12. Method for making a reusable belt according to one or more of claims 1 to 11, comprising the step a) of conforming to size said central portion (10), said first side portion (11) and said second side portion (12), wherein said central portion (10) is made of a material having elastic capabilities lower than those of the material of which said first side portion (11) and/or said second side portion (12) is/are made.

13. Method according to claim 12, **characterised in that** said step a) comprises the step a1) of starting from a single piece of a first fabric (40) and the step a2) of cutting to size said single piece of first fabric (40) to obtain said central portion (10), said first side portion (11) and said second side portion (12), and **in that** it comprises the step b) of integrally combining said second fabric (41) with said first fabric (40), at said central portion (10).

14. Method according to claim 12, **characterised in that** said step a) comprises the step a1') of starting from three distinct pieces of a first fabric and the step a2') of cutting to size each piece of said three pieces of said first fabric to obtain said central portion (10), said first side portion (11) and said second side portion (12), and **in that** it comprises the step b) of integrally combining said second fabric (41) with said first fabric (40), at said central portion (10), and the step d) of integrally constraining said first side portion (11) and/or said second side portion (12) to said first edge (10a) and/or to said second edge (10b) of said central portion (10), along the respective side connecting edge (11a, 12a).

15. Method according to claim 13 or 14, **characterised in that** said step b) and/or said step d) comprise/comprises the step c) of sewing, respectively, said second fabric (41) to said first fabric (40) and/or said first side portion (11) and/or said second side portion (12) to said central portion (10).

16. Diaper (100) comprising an absorbent article (2) and a reusable belt (1) according to one or more of claims 1 to 11, said reusable belt (1) being reversibly combinable with said absorbent article (2).

17. Diaper (100) according to claim 16, **characterised in that** said absorbent article (2) comprises a permeable upper web 50, a waterproof lower web (51), an absorbent core (52) comprising absorbent material (53) arranged between an upper layer (53) and a lower layer (54), said absorbent core (52) being arranged between said upper web (50) and said lower web (51), wherein the absorbent core (52) preferably comprises one or more attaching areas (30) in which the upper layer (50) is combined with the lower layer (51), said one or more attaching areas being devoid of said absorbent material.

18. Diaper according to claim 16 or 17, **characterised in that** said absorbent article (2) comprises at least one adhesive band (60) arranged on the outer surface (51a) of said waterproof lower web (51), said at least one adhesive band (60) being covered by at least one removable protection strip (61).
